# EUROPEAN PATENT SPECIFICATION

(11) **EP 3 858 315 B1**
(45) Date of publication and mention of the grant of the patent: **20.12.2023**
(21) Application number: 19867760.1
(22) Date of filing: 05.09.2019
(51) Int. Cl.: A61F 13/511

(54) **ABSORBENT ARTICLE**
ABSORBIERENDER ARTIKEL
ARTICLE ABSORBANT

(30) Priority: 28.09.2018 JP 2018183441
(43) Date of publication of application: 04.08.2021
(73) Proprietor: Kao Corporation, Chuo-ku, Tokyo 103-8210 (JP)
(72) Inventor: TOMITA, Mina, Haga-gun, Tochigi 321-3497 (JP); KOUTA, Takuya, Haga-gun, Tochigi 321-3497 (JP)
(74) Representative: Vossius & Partner Patentanwälte Rechtsanwälte mbB
(86) International application number: PCT/JP2019/035018
(87) International publication number: WO 2020/066530

(56) References cited:
- WO-A1-2014/050414
- WO-A1-2017/094409
- GB-A- 2 559 703

## Description

### Technical Field

The present invention relates to an absorbent article.

### Background Art

In the field of absorbent articles, such as disposable diapers and sanitary napkins, an improvement on a topsheet that is to come into contact with the skin of a wearer have been made in an attempt to improve wearer comfort. For instance, JP 2003-220660 A listed below proposes using as a topsheet a complex nonwoven fabric composed of spun-bonded nonwoven and melt-blown nonwoven. According to JP 2003-220660 A, the feel to the touch of the topsheet is improved by using the spun-bonded nonwoven side of the complex nonwoven fabric as a skin contact surface and allowing the surface of the spun-bonded nonwoven fabric to have an average friction coefficient and a mean deviation of the friction coefficient within respective specific ranges.

Apart from the above described technique, the applicant common to this application has proposed using as a topsheet a dual layer laminate sheet having a three-dimensional structure with the aim of reducing the friction against the wearer's skin to improve wearer comfort while providing an appealing appearance (see JP 2017-104501 A below). According to this technique, the topsheet is soft, and the upper layer thereof is prevented from peeling and lifting off the lower layer during wear of the absorbent article.

### Summary of Invention

The invention relates to an absorbent article as defined in independent claim 1. Preferred aspects are defined in the dependent claims.

### Brief Description of Drawings

[Fig. 1] Fig. 1 is a partially cutaway perspective view illustrating one embodiment of the topsheet used in the absorbent article of the present invention.
[Fig. 2] Fig. 2(a) is a schematic cross-sectional view illustrating another embodiment of the topsheet used in the absorbent article of the invention before rubbing, and Fig. 2(b) is a schematic cross-sectional view of the topsheet after rubbing.
[Fig. 3] Fig. 3 is a diagram illustrating a method for producing the topsheet shown in Fig. 2(a).
[Fig. 4] Fig. 4 is a diagram illustrating a method for determining the shape of a bond.
[Fig. 5] Fig. 5(a) is a schematic cross-sectional view illustrating still another embodiment of the topsheet used in the absorbent article of the invention before rubbing, and Fig. 5(b) is a schematic cross-sectional view of the topsheet after rubbing.
[Fig. 6] Fig. 6 is a diagram illustrating a method for producing the topsheet shown in Fig. 5(a).
[Fig. 7] Fig. 7(a) is a schematic cross-sectional view illustrating yet another embodiment of the topsheet used in the absorbent article of the invention before rubbing, and Fig. 7(b) is a schematic cross-sectional view illustrating yet another embodiment of the topsheet before rubbing.
[Fig. 8] Fig. 8(a) is a schematic view showing an essential part of a roller used for producing the topsheet shown in Fig. 7(a), and Fig. 8(b) is that for producing the topsheet shown in Fig. 7(b).
[Fig. 9] Fig. 9(a) and Fig. 9(b) are each a plan view showing an arrangement pattern of the bonds of the topsheet shown in Fig. 7(b).

### Description of Embodiments

When bodily fluids, such as urine and sweat, are discharged onto an absorbent article worn by a wearer, the moisture content of the skin of the wearer may increase. The increased moisture content of the skin contributes to an increase in frictional force against the topsheet. This may cause rubbing of the skin, which can lead to skin troubles. JP 2003-220660 A and JP 2017-104501 A do not discuss reducing the frictional force between the topsheet and the wet skin.

The present invention relates to an absorbent article that is gentle to the skin, the absorbent article having a reduced frictional force even when the skin has an increased moisture content during wear of the absorbent article.

The invention will be described on the basis of its preferred embodiments with reference to the accompanying drawings. The absorbent article of the invention generally has an oblong shape, i.e., having a longitudinal direction that corresponds to the front-to-back direction of the wearer (namely, the direction extending from the abdomen side to the back side via the crotch of the wearer) and a lateral direction perpendicular to the longitudinal direction. The absorbent article has a crotch portion to be worn about the crotch of a wearer, and a front portion and a rear portion extending forward and rearward of the crotch portion, respectively. The crotch portion has a target zone that is to face a point of body fluid discharge while worn. The target zone is usually located in the longitudinal middle of the absorbent article or in the vicinity thereof.

An absorbent article generally includes a topsheet on the skin facing side, a backsheet on the non-skin facing side, and an absorbent member interposed between the two sheets. The topsheet may be a liquid-permeable sheet, such as nonwoven fabric or perforated plastic film. The topsheet may have a three-dimensionally shaped surface on its skin facing side. For example, the topsheet may have a plurality of projections discretely distributed on its skin facing side. The topsheet may have alternating ridges and furrows extending in one direction on the skin facing surface side thereof. To provide such a three-dimensional configuration, the topsheet may be formed of two or more sheets of nonwoven fabric.

The back sheet may be, e.g., sparingly liquid-permeable film or a spun-bonded/melt-blown/spun-bonded complex nonwoven fabric. A sparingly liquid permeable film having been rendered water vapor permeable by making a plurality of micropores may also be useful. With a view to further improving the feel of the absorbent article, a sheet material having a good hand, such as nonwoven fabric, may be laminated on the outer surface of the back sheet.

The absorbent member includes an absorbent core. The absorbent core may be made of airlaid hydrophilic fibers, such as cellulose fibers exemplified by pulp fiber, an airlaid mixture of hydrophilic fibers and an absorbent polymer, a layer of an absorbent polymer, or a stacked sheet structure composed of two absorbent sheets having an absorbent polymer sandwiched therebetween. The absorbent core may be covered at least on its skin facing side with a liquid permeable core wrap sheet or covered on its entire surface including the skin and non-skin facing sides with a core wrap sheet. The core wrap sheet may be a tissue layer made of hydrophilic fibers or liquid permeable nonwoven fabric.

The absorbent article may further include, in addition to the topsheet, backsheet and absorbent member, a pair of leak barrier cuffs extending in the longitudinal direction on either lateral side portion on the skin facing side thereof, depending on the specific use of the absorbent article. The leak barrier cuff generally has a proximal edge and a free, distal edge. The leak barrier cuff has the proximal edge fixed to the skin facing side of the absorbent article and stands up away from the skin facing side. The leak barrier cuff is made of a liquid-resistant or water-repellent and air-permeable material. An elastic member, such as a rubber thread, may be arranged in a stretched state along the free distal edge of the leak barrier cuff or the vicinity thereof. On contraction of the elastic member during wear of the absorbent article, the cuff rises toward the wearer's body so that a bodily fluid discharged on the topsheet may be effectively prevented from running and leaking laterally.

The absorbent article may further have a pressure-sensitive adhesive layer on the surface of the non-skin facing side, with which the absorbent article can be secured to underwear or another absorbent article on use.

Examples of the absorbent article having the above configuration include, but are not limited to, open-style disposable diapers, pull-on disposable diapers, sanitary napkins, and incontinence pads.

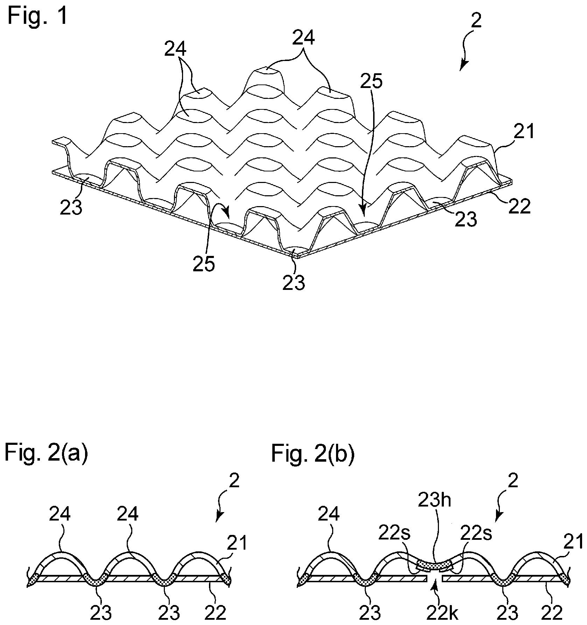

Fig. 1 illustrates an example of the topsheet used in the absorbent article of the invention. The illustrated topsheet 2 is in a state before use of the absorbent article. "Before use" also means "before rubbing or being rubbed" under the conditions described later. The topsheet 2 is a laminate sheet composed of a skin contact side sheet 21 and a non-skin contact side sheet 22 united to each other at a plurality of bonds 23. The skin contact side sheet 21 forms a large number of projections 24 protruding away from the non-skin contact side sheet 22 in portions other than the bonds 23. Thus, the skin contact side sheet 21, which defines the skin contact side of the topsheet 2, has an undulating three-dimensional structure. On the other hand, the non-skin contact side of the topsheet 2 is substantially flat.

Referring to Fig. 1, the projection 24 is formed by the skin contact side sheet 21 bulging outward at locations between the bonds 23. The inside of the projection 24 is not filled with the material making the topsheet 2 but provides a space. That is, the projection 24 is hollow. The projections 24 are regularly discretely arranged over the entire area of the skin contact side of the topsheet 2. The bond 23 located between adjacent projections 24 defines the bottom of a recess 25 of the three-dimensional structure of the topsheet 2. The bonds 23 are usually formed by debossing the skin contact side sheet 21 and the non-skin contact side sheet 22 in superposed relation with or without heat or by ultrasonic debossing.

The topsheet 2 having such a three-dimensional structure can be produced by using, for example, the apparatus illustrated in Fig. 2 of JP 2005-111908A. The apparatus includes a first roller and a second roller, the contour of the peripheral surface of the former and that of the latter being capable of meshing with each other, and these two rollers are arranged in mesh in the apparatus. The apparatus further includes an anvil roller arranged in contact with the peripheral surface of the first roller. A web of skin contact side sheet 21 is introduced into the nip of the intermeshing first and second rollers to three-dimensionally deform the skin contact side sheet 21. A web of non-skin contact side sheet 22 is fed so as to be superposed on the deformed skin contact side sheet 21 while the skin contact side sheet 21 is held to the peripheral surface of the first roller to keep the three-dimensional deformation. The sheets superposed on each other are passed and pressed between the first roller and the anvil roller with or without heat. The skin contact side sheet 21 and the non-skin contact side sheet 22 are thus press bonded together in parts between a pair of rollers to make a plurality of bonds 23.

The topsheet 2 has a region where, when rubbed under the following conditions, a friction coefficient (hereinafter referred to as MIU) or a mean deviation of the friction coefficient (hereinafter referred to as MMD) decreases by 5% or more or 15% or more, respectively, compared to before the rubbing. The above specified region will hereinafter be referred to as a friction decreasing region.

### Conditions of rubbing:

A collagen film (EDCOL-R, available from PRIRO) cut into a square of 10 cm is immersed in ion-exchanged water, e.g., for 1 minute, taken out, and dewatered to prepare a wet collagen film. The water content of the wet collagen film is 100 to 150 mass% relative to its dry weight. The dewatering, i.e., removal of excess water is achieved by, for example, placing the collagen film between two wipes (Kim Towel, from Nippon Paper Crecia Co., Ltd.) under a load of 3.5 kPa for 1 minute.

A 1.5 kg weight with a 7 cm square base is provided, and the wet collagen film is fixed to the base of the weight. The weight having the collagen film attached thereto is put on the topsheet of an absorbent article resting on a flat hard surface and reciprocated 10 times in the longitudinal direction of the absorbent article under a load of 3.0 kPa at 23°C and 50% RH. Preferably, the longitudinal direction coincides with the machine direction (MD). The moving distance of the weight may be decided according to the size of the topsheet, but is preferably set to 5 to 15 cm from in view of performing a highly reproducible measurement. The weight can be reciprocated either manually or mechanically. The moving speed is preferably 2 to 3 cm/s in view of making a highly reproducible measurement.

The rubbing under the above specified conditions is a simulation of the friction between the topsheet 2 and a wet skin having an increased moisture content as a result of discharge of bodily fluids, such as urine and sweat, during wear of an absorbent article. Since the absorbent article includes the topsheet having the friction decreasing region, the frictional force between the skin and the topsheet 2 can be reduced even when the moisture content of the skin increases during use of the absorbent article, thereby preventing skin problems from arising. The friction decreasing region decrease in at least one of MIU and MMD when rubbed. It is preferable that the friction decreasing region decrease in both MIU and MMD when rubbed.

In view of reducing the frictional force of the topsheet 2 against the skin, preventing skin problems, and maintaining an aesthetic appearance of the topsheet 2, the topsheet 2 preferably has the friction decreasing region at any arbitrarily selected locations, not localized in a specific area or at a specific location. It is particularly preferred for the topsheet 2 to have the friction decreasing regions distributed over the entire area thereof. In this case, it is preferable that the friction decreasing regions be arranged discretely over the entire area of the topsheet 2.

The friction decreasing region shows a 5% or more reduction in MIU or a 15% or more reduction in MMD as a result of the rubbing under the above conditions. In view of friction reduction under the wet condition, the MIU is preferably reduced by 10% or more, and the MMD is preferably reduced by 30% or more. In view of preventing diaper from sliding out of position, the reduction in MIU is preferably not more than 60%, more preferably not more than 50%, and the reduction in MMD is preferably not more than 70%, more preferably not more than 60%.

The closer an MIU value to 0, the lower the frictional force. In this respect, the MIU of the friction decreasing region before the rubbing under the above conditions is preferably 0.30 or less, more preferably 0.28 or less. The lower limit of the MIU is about 0.15. The MIU of the friction decreasing region after the rubbing under the above conditions is preferably 0.27 or less, more preferably 0.25 or less. The lower limit of the MIU after the rubbing is about 0.10. The change in MIU of the friction decreasing region before and after the rubbing in terms of the ratio of the MIU after the rubbing to the MIU before the rubbing, i.e., MIU after rubbing/MIU before rubbing, is preferably 0.95 or lower, more preferably 0.90 or lower.

Regarding MMD, the closer an MMD value to 0, the lower the frictional force. In this respect, the MMD of the friction decreasing region before the rubbing under the above conditions is preferably 0.015 or less, more preferably 0.012 or less. The lower limit of the MMD is about 0.005. The MMD of the friction decreasing region after the rubbing under the above conditions is preferably 0.010 or less, more preferably 0.007 or less. The lower limit of the MMD after the rubbing is about 0.003. The change in MMD of the friction decreasing region before and after the rubbing in terms of the ratio of the MMD after the rubbing to the MMD before the rubbing, i.e., MMD after rubbing/MMD before rubbing, is preferably 0.85 or less, more preferably 0.70 or less.

Measurement of MIU and MMD of the topsheet 2 before and after the rubbing can be carried out using a surface tester KES-FB4 from Kato Tech Co., Ltd. The measurement is made on the topsheet 2 as is incorporated in the absorbent article. The contact sensor of the tester is brought into contact with the skin contact side of the topsheet 2. The contact sensor is moved 3 cm in a horizontal plane at a constant speed of 0.1 cm/sec while applying a load of 5 kPa (50 gf/cm²) to the topsheet 2. The moving direction of the contact sensor is the longitudinal direction of the absorbent article. The MIU and MMD are measured at three points within a movement area, and the respective average values are taken as the MIU and MMD of the topsheet 2.

It is preferred that the friction between the topsheet 2 after the rubbing and a wet collagen film decrease by 40% or more, more preferably 50% or more, with respect to the friction between the topsheet 2 and a wet collagen film before the rubbing. The friction against the wet collagen film is measured as follows.

### Method for measuring friction against wet collage film:

A collagen film (EDCOL-R, available from PRIRO) cut into a 2.5 cm square is immersed in ion-exchanged water, e.g., for 1 minute, taken out, and dewatered to prepare a wet collagen film. The moisture content of the wet collagen film is 100 to 150 mass% relative to its dry weight. The dewatering, i.e., removal of excess water is achieved by, for example, placing the collagen film between two wipes (Kim Towel, from Nippon Paper Crecia Co., Ltd.) under a load of 3.5 kPa for 1 minute.

A 10 cm square is cut out from the topsheet of an absorbent article such that one side of the square is parallel to and 1 cm away from the longitudinal end of the absorbent article. The cut sheet is fixed to an acrylic plate.

The wet collagen film is attached intimately to the measurement head of Friciometer FR700, from Courage + Khazaka electronic GmbH, and the head with the collagen film attached thereto is attached to the probe. The probe is vertically applied onto the topsheet to measure the friction under the following conditions. Measurement is made at 23°C, 50% RH.
- Rotational speed: 50 rpm
- Measurement area: Φ 16mm (Teflon^{®} head)
- Pressure: 0.7 N (3.5 kPa)
- Measurement interval: 1 second.

The average value for 20 seconds (20 points) between 10 seconds and 30 seconds from the start of rotation is taken as a found value. The same measurement is conducted at three different positions, and the average of the three found values is taken as the friction (Frictiometer value) against the wet collagen film.

Measurement of the friction against a dry collagen film is carried out in the same manner, except for using a non-moistened commercially available collagen film (EDCOL-R, available from PIRRO) instead of the wet collagen film. The friction of the topsheet before the rubbing is also measured in the same manner as described above.

In order for the friction decreasing region to decrease in MIU or MMD as a result of the rubbing under the above conditions, it is advantageous that the skin contact side sheet 21 and the non-skin contact side sheet 22, which constitute the topsheet 2, be peelable from each other at the bond 23 on being rubbed under the above conditions. A region in which the skin contact side sheet 21 is separate from and the non-skin contact side sheet 22 is created by the peeling of these two sheets due to the rubbing. As a result, the range of movement of the skin contact side sheet 21 is expanded, thereby reducing the resistance to friction. In addition, variations in frictional force also reduce. With the view to reducing the frictional force, it is preferred to appropriately adjust the bonding strength between the skin contact side sheet 21 and the non-skin contact side sheet 22 at the bonds 23. The bonding strength is adjusted by, for example, controlling the pressure between the first roller 11 and the anvil roller 14 and the heating temperature in the apparatus illustrated in Fig. 2 of JP 2005-111908A cited above. Specifically, weaker bonding of the two sheets 21 and 22 at the bond 23 than usual can be made by reducing the pressure between the first roller 11 and the anvil roller 14 or lowering the set temperature of the heating roller. Such weak bonding can promote peel-apart separation between the two sheets by rubbing.

To allow the skin contact side sheet 21 to separate from the non-skin contact side sheet 22 by rubbing, it is advantageous that the bonding strength of the bond 23 be relatively low as discussed above. However, if the bonding strength is too low, peel-apart separation of the two sheets 21 and 22 may occur unintentionally, which can seriously deteriorate the appearance of the topsheet 2. Therefore, the friction decreasing region is preferably designed such that the skin contact side sheet 21 and the non-skin contact side sheet 22 may peel from each other when a shear force of 10 N or higher is applied by rubbing. In view of further preventing unintended separation between the skin contact side sheet 21 and the non-skin contact side sheet 22, it is more preferable that the friction decreasing region be designed such that peel-apart separation may occur when a shear force of 18 N or higher, even more preferably 20 N or higher is applied. To surely reduce the MIU or MMD of the friction decreasing region by rubbing, the peel-apart separation is preferably designed to occur when a shear force of 27 N or less, more preferably 25 N or less, even more preferably 23 N or less is applied.

The shear strength of the topsheet 2 is measured by the following method.

A specimen including the friction decreasing region is cut out of a topsheet 2. The specimen has a rectangular shape having a length of 50 mm in the longitudinal direction and a width of 30 mm in the lateral direction in plan view. Separately, a double-sided adhesive tape (No. 500, from Nitto Denko Corp.) of the same shape and size as the specimen is stuck on a PET film (Star OHP Film, from Star Corp.) to prepare laminate (A). The specimen is stuck on the adhesive tape of laminate (A) with the non-skin contact side sheet 22 facing the adhesive tape to obtain a laminate (B), which is rolled back and forth five times with a 1 Kg roller to ensure firm adhesion of the specimen and the adhesive tape in laminate (B).

As another adherend, a male component of a mechanical fastener (1600PPI, available from 3M) having a shape and size enough to cover the entire area of the specimen is provided. This adherend is attached to the skin contact side sheet 21 of the specimen of laminate (B) with the hook side of the adherend facing the skin contact side sheet 21, followed by pressing with a load of 1.5 kPa. The resulting specimen assembly is set on jaws of a tensile tester (Augograph AG-X, from Shimadz Corp.) at an initial jaw spacing of 150 mm, and 180 degree peel test is performed. In the test, one of the longitudinal ends of laminate (B) is clamped into one of the jaws of the tester and the adherend at the other end into the other jaw, and the specimen assembly is pulled at 180° in opposite directions at a tension rate of 300 mm/min. The maximum tensile load exerted is recorded. For a topsheet, the above specimen is taken from the laterally left, middle, and right portions in each of the front, crotch, and rear portions of an absorbent article to make a total of nine specimen assemblies. The average of the nine found values is defined as the average shear strength of the topsheet. The found values of specimen assemblies that are less than 75% of the above defined average shear strength are averaged, and the resulting average is defined as the shear strength of the friction decreasing region. When there is no found value less than 75%, the shear strength of the topsheet is taken as the shear strength of the friction decreasing region. The same measurement is made on a total of three topsheets, and the average value of the resulting three found values is defined as the shear value of the friction decreasing region. When it is not possible to prepare nine specimens per topsheet, six or four specimens may be cut out from the above described locations except either the crotch portion or the lateral middle portions or both, and the average of the six or four found values is taken as the average shear value of the topsheet.

Fig. 2(a) illustrates another embodiment of the topsheet 2 used in the invention. As illustrated, the bonds 23 between the skin contact side sheet 21 and the non-skin contact side sheet 22 in the friction decreasing region have a downward convex shape in cross section taken in the thickness direction of the topsheet 2. Due to the bond 23 having such a downward convex shape, the fibers making up the topsheet 2 are ready to be severed around the bond 23 when the topsheet 2 is rubbed under the above conditions, and as a result, the skin contact side sheet 21 and the non-skin contact side sheet 22 are easily separated. In particular, the fibers making up the non-skin contact side sheet 22 are easily severed. When separation occurs between the skin contact side sheet 21 and the non-skin contact side sheet 22 in such a manner, a through-hole 22k will easily be formed in the non-skin contact side sheet 22 at the location where the bond 23 has been, as illustrated in Fig. 2(b). Formation of the through-holes 22k in the non-skin contact side sheet 22 is advantageous in terms of improving the liquid permeability of the topsheet 2.

Furthermore, when the constituent fibers of the topsheet 2, particularly the constituent fibers of the non-skin contact side sheet 22 are severed around the bond 23, it is likely that fibers 22s remain held to the skin contact side sheet 21 as illustrated in Fig. 2(b). Consequently, in the skin contact side sheet 21, a portion 23h resulting from the separation of the bond 23 has an increased fiber density compared with the surroundings of the portion 23h. This creates a gradient in the capillary force. Furthermore, the portion 23h still has a downward convex shape like the bond 23. As a result of these factors acting in combination, the topsheet 2 after the skin contact side sheet 21 and the non-skin contact side sheet 22 are peeled apart by rubbing exhibits improved absorption for liquid droplets present on the skin contact side sheet 21.

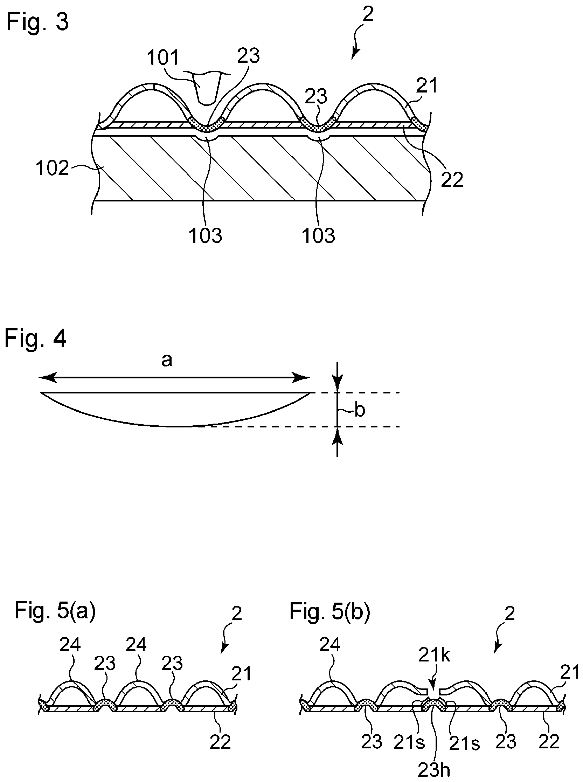

The topsheet 2 shown in Fig. 2(a) may be produced by, for example, using a combination of an anvil roller 102 and an engraved roller (not shown) having projections 101 protruding toward the anvil roller 102 in conformity to the shape of the bonds 23, as illustrated in Fig. 3. The anvil roller 102 used in this method has on its peripheral surface recesses 103 at locations corresponding to the projections 101 of the engraved roller. The use of so configured combination of rollers enables successful formation of the bonds 23 having a downward convex shape.

The shape of the bond 23 in the topsheet 2 shown in Fig. 2(a) is determined by microscopically observing cut planes of the bonds 23, each cut plane passing through the center of a bond 23 in the machine direction (MD) or the cross-machine direction (CD) of the topsheet 2. The cut plane of a bond 23 is observed to measure values a and b (see Fig. 4), and such observation is made on ten bonds 23 for each of the MD and CD. When the average of the found values of the ratio b/a is 0.05 or greater, the bonds 23 are regarded to have a downward convex shape. The shape of the bonds 23 shown in Fig. 5(a), which will be discussed below, is determined in the same manner.

Fig. 5(a) illustrates still another embodiment of the topsheet 2 used in the invention. As illustrated, the bonds 23 between the skin contact side sheet 21 and the non-skin contact side sheet 22 in the friction decreasing region have an upward convex shape in cross section taken in the thickness direction of the topsheet 2. Due to the bond 23 having such an upward convex shape, the fibers making up the topsheet 2 are ready to be severed around the bond 23 when the topsheet 2 is rubbed by the wearer's skin while worn, and as a result, the skin contact side sheet 21 and the non-skin contact side sheet 22 are easily separated. In particular, the fibers constituting the skin contact side sheet 21 are easily severed. When separation occurs between the skin contact side sheet 21 and the non-skin contact side sheet 22 in such a manner, a through-hole 21k will easily be formed in the skin contact side sheet 21 at the location where the bond 23 has been, as illustrated in Fig. 5(b). Formation of the through-hole 21k in the skin contact side sheet 21 is advantageous in terms of permitting feces, such as loose stool containing solid matter, to easily pass through the topsheet via the through-hole 21k.

Furthermore, when the constituent fibers of the topsheet 2, particularly the constituent fibers of the skin contact side sheet 21 are severed around the bond 23, it is likely that fibers 21s remain held to the non-skin contact side sheet 22 as illustrated in Fig. 5(b). Consequently, in the non-skin contact side sheet 22, a portion 23h resulting from the separation of the bond 23 has an increased fiber density as compared with the surroundings of the portion 23h. This creates a gradient in the capillary force. As a result, the water content of the feces, such as loose stool, that has passed through the through-hole 21k of the skin contact side sheet 21 is allowed to easily pass through the portions 23h of the non-skin contact side sheet 22.

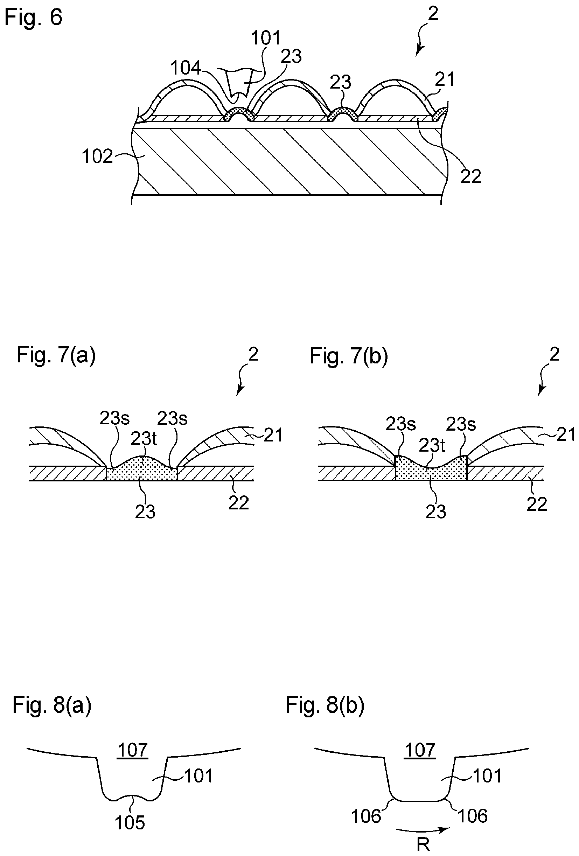

The topsheet 2 shown in Fig. 5(a) may be produced by, for example, using a combination of an anvil roller 102 and an engraved roller (not shown) having projections 101 the top face of which has a recess 104 in conformity to the shape of the bonds 23, as illustrated in Fig. 6. The use of so configured combination of rollers enables successful formation of the bonds 23 having an upward convex shape.

Figs. 7(a) and 7(b) illustrates the topsheet 2 used in the invention. As illustrated, with a focus on a single bond 23 between the skin contact side sheet 21 and the non-skin contact side sheet 22 in the friction decreasing region, the thickness of the bond 23 varies from one location to another in cross section taken in the thickness direction of the topsheet 2. More specifically, the bond 23 shown in Fig. 7(a) has a larger thickness in the central portion 23t than in the peripheral portion 23s, and the bond 23 shown in Fig. 7(b) has a larger thickness in the peripheral portion 23s than in the central portion 23t. When a single bond 23 includes portions having different thicknesses as described, rubbing-induced separation between the skin contact side sheet 21 and the non-skin contact side sheet 22 is initiated from between the portions having different thicknesses. Thus, the difference in thickness of the bond 23 is advantageous in that peel-apart separation starts preferentially from between the portions having different thicknesses. In particular, when the bond 23 of Fig. 7(a) has a shape elongated in the longitudinal direction of the absorbent article, peel-apart separation is advantageously apt to start from the longitudinal ends of the bond 23 when the topsheet is rubbed in the longitudinal direction.

The bond 23 having the shape shown in Fig. 7(a) may be formed by using a combination of an anvil roller (not shown) and a roller 107 having a projection 101 the top face of which has a recess 105 in conformity to the shape of the bond 23 as shown in Fig. 8(a). The bond 23 having the shape shown in Fig. 7(b) may be formed by using a combination of an anvil roller (not shown) and a roller 107 having a projection 101 as shown in Fig. 8(b). The projection 101 has its leading and trailing edges 106 in the rotational direction R of the roller 107, and these edges 106 are rounded smoothly to have a rounded shape.

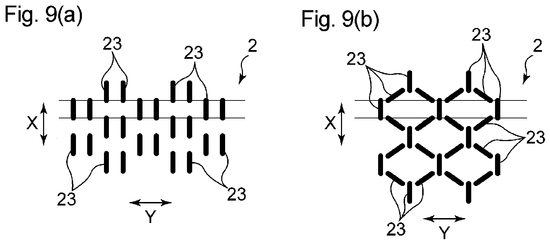

In the formation of the bonds 23 having the shape illustrated in Fig. 7(b), it is advantageous to arrange the bonds 23 in a well-thought-out pattern. For example, the bonds 23 are preferably arranged such that, when the top sheet 2 is viewed from the side, both ends in the longitudinal direction X of one bond 23 overlap the longitudinal ends of another bond 23 or other bonds 23 adjacent thereto in the lateral direction Y of the bond 23 as illustrated Figs. 9(a) and 9(b). The arrangement patterns of the bonds 23 shown in Figs. 9(a) and 9(b) correspond to the arrangement patterns of the projections 101 provided on the roller 107 (Figs. 8(a) and 8(b)) used to form the bonds 23. Therefore, in the transverse cross section of the roller 107, the leading and trailing ends, in the rotational direction, of the projection 101 overlap the leading and trailing ends, in the rotational direction, of another projection 101 adjacent in the axial direction. When the bonds 23 are formed using the roller 107 having the projections 101 in the above arrangement, the linear pressure applied by the leading and trailing ends, in the rotational direction, of the individual projections 101 is lower than that applied by the central portion of the top face of the projections 101. Thus, the bonds 23 having the central portion 23t with a relatively small thickness and the peripheral portion 23s with a relatively large thickness can easily be formed.

In each of the above embodiments, it is advantageous to use an absorbent member having a high basis weight portion and a low basis weight portion. When absorbing liquid, the absorbent member having a high basis weight portion and a low basis weight portion shows various increases in thickness depending on the basis weight. Therefore, the absorbent member after liquid absorption applies different pressures toward the wearer's skin depending on the basis weight. Because a higher frictional force is applied to the region of the topsheet 2 corresponding to the region of the absorbent member exerting a higher pressure toward the wearer's skin, peel-apart separation between the skin contact side sheet 21 and the non-skin contact side sheet 22 occurs preferentially in that region of the topsheet. As a result, the MIU or MMD of the topsheet 2 can be selectively reduced.

The skin contact side sheet 21 and the non-skin contact side sheet 22 composing the topsheet 2 used in the above embodiments may be a fiber sheet, such as nonwoven fabric, woven fabric, or knitted fabric, or a plastic film. A fiber sheet is preferred in terms of feel to the touch, and nonwoven fabric is particularly preferred. The sheet materials making up the skin contact side sheet 21 and the non-skin contact side sheet 22 may be the same or different.

Examples of the nonwoven fabric include through-air, spun-bonded, hydroentangled, melt-blown, resin-bonded, and needle-punched nonwovens. A complex nonwoven fabric composed of two or more different nonwoven fabric webs or a laminated nonwoven fabric composed of a nonwoven fabric web and a plastic film may also be used. Preferred of them is a through-air or spun-bonded nonwoven fabric. The nonwoven fabric preferably has a basis weight of 10 g/m² or more, more preferably 15 g/m² or more, and preferably 40 g/m² or less, more preferably 35 g/m² or less.

The fibers making up the nonwoven fabric may be made of various thermoplastic resins. Examples of the thermoplastic resin include polyolefins, such as polyethylene and polypropylene, polyesters, such as polyethylene terephthalate, polyamides, such as nylon 6 and nylon 66, polyacrylic acid, poly(alkyl methacrylate), polyvinyl chloride, and polyvinylidene chloride. These resins may be used either individually or in combination of two or more thereof in the form of a polyblend or conjugate fibers, such as sheath/core or side-by-side conjugate fibers.

While the invention has been described on the basis of its preferred embodiments, it should be understood that the invention is not limited thereto, and alterations can be made therein.

For example, the above embodiments relate to application of the invention to a topsheet, but the invention can also be applied to a sheet defining a skin contact surface other than the topsheet. Examples of such a sheet include a sheet forming a leak barrier cuff.

In the above-described embodiments, a laminate sheet having a three-dimensional structure has been described as an example of a sheet whose MIU or MMD decreases by rubbing, but other sheets may be used.

In the above embodiments, the purpose of lowering MIU or MMD by rubbing is accomplished by peel-apart separating two sheets of a laminate sheet at the bonds, but MIU or MMD may be lowered by other means.

### Examples

The invention will now be illustrated in greater detail by way of Examples, but it should be understood that the invention is not deemed to be limited thereto.

### Example 1

A laminate sheet having a three-dimensional structure shown in Fig. 1 was made using the apparatus illustrated in Fig. 2 of JP 2005-111908A. The laminate sheet was used as a topsheet. The first roller 11 of the apparatus was not heated, and, instead, the temperature of the anvil roller 14 was set to 175°C. The laminate sheet had the friction decreasing regions arranged discretely over the entire area thereof. The skin contact side sheet 21 and the non-skin contact side sheet 22 of the topsheet were each formed of a through-air nonwoven fabric having a basis weight of 18 g/m² and made up of sheath/core conjugate fibers having polyethylene as a sheath component and polyethylene terephthalate as a core component.

An open-style, medium size disposable diaper Merries^{®} from Kao Corp. was used, with its topsheet replaced with the above prepared laminate sheet. The topsheet of the so modified diaper was rubbed under the above conditions, and MIU and MMD before and after the rubbing were measured by the above-mentioned method. The results obtained are shown in Table 1 below. Additionally, the friction against a dry collagen film and that against a wet collagen film before and after the rubbing were measured by the above-described method.

### Comparative Example 1

A laminate sheet and a disposable diaper were prepared in the same manner as in Example 1, except for raising the set temperature of the anvil roller 14 to 220°C. MIU and MMD of the topsheet of this diaper were measured before and after the rubbing by the above-described method. The results are shown in Table 1.

**Table 1**

| | | Before Rubbing | After Rubbing | After Rubbing /Before Rubbing | Percent Decrease |
|---|---|---|---|---|---|
| Example 1 | MIU | 0.25 | 0.22 | 0.88 | 12% |
| | MMD | 0.0094 | 0.0064 | 0.68 | 32% |
| | Friction against Dry Collagen Film | 82.3 | 79.5 | 0.97 | 3% |
| | Friction against Wet Collagen Film | 258.8 | 108.5 | 0.42 | 58% |
| | Increase (%) in Friction due to Wetting | 314% | 136% | 0.43 | 57% |
| Comparative Example 1 | MIU | 0.25 | 0.24 | 0.96 | 4% |
| | MMD | 0.0092 | 0.0082 | 0.89 | 11% |
| | Friction against Dry Collagen Film | 82.3 | 81.9 | 0.99 | 1% |
| | Friction against Wet Collagen Film | 258.8 | 255.4 | 0.99 | 1% |
| | Increase (%) in Friction due to Wetting | 314% | 312% | 0.99 | 1% |

As is clear from the results in Table 1, the diaper of Example 1 has a lower MIU and a lower MMD after the rubbing than the diaper of Comparative Example 1. In addition, it is seen that the diaper of Example 1 has reduced friction against the wet collagen film, which is simulated skin, after the rubbing.

### Industrial Applicability

The invention provides an absorbent article that has a reduced frictional force against the skin while worn even when the skin has an increased moisture content and is therefore gentle to the skin.

## Claims

1. An absorbent article comprising, on a skin contact side thereof, a sheet (2) having a region where, when rubbed under the conditions below, a friction coefficient or a mean deviation of the friction coefficient decreases by 5% or more or 15% or more, respectively, compared to before the rubbing, and having a longitudinal direction corresponding to a front-to-back direction of a wearer and a lateral direction perpendicular to the longitudinal direction, wherein the sheet is a three-dimensionally shaped laminate sheet composed of a skin contact side sheet (21) and a non-skin contact side sheet (22), the skin contact side sheet (21) and the non-skin contact side sheet (22) being united to each other at a plurality of bonds (23), the skin contact side sheet (21) forming a plurality of projections (24) protruding away from the non-skin contact side sheet (22) in portions other than the bonds (23), and the region being designed such that the skin contact side sheet (21) and the non-skin contact side sheet (22) peel from each other at the bond (23) when rubbed, wherein the bond (23) in the region comprises portions having different thicknesses, and peel-apart separation between the skin contact side sheet (21) and the non-skin contact side sheet (22) is designed to initiate from between the portions having different thicknesses by the rubbing:
conditions: a wet collagen film is reciprocated 10 times on the sheet (2) in the longitudinal direction of the sheet (2) under a load of 3.0 kPa; and the wet collagen film has a water content of 100 to 150 mass% relative to a dry weight thereof and is prepared by immersing a collagen film in ion-exchanged water for 1 minute, taking out the collagen film, and removing excess water;
wherein the friction coefficient and the mean deviation of the friction coefficient are measured according to the method of the description.

2. The absorbent article according to claim 1, wherein the sheet (2) is a liquid-permeable topsheet (2) defining the skin contact side, and the region is present in at least a laterally middle portion of the topsheet (2).

3. The absorbent article according to claim 1 or 2, wherein the bond (23) in the region has a downward convex shape in cross-section taken in the thickness direction, and fibers making up the non-skin contact side sheet (22) are ready to be severed around the bond when rubbed.

4. The absorbent article according to claim 1 or 2, wherein the bond (23) in the region has an upward convex shape in cross-section taken in the thickness direction, and fibers making up the skin contact side sheet (21) are ready to be severed around the bond when rubbed.

5. The absorbent article according to any one of claims 1 to 4, wherein the region is designed such that the skin contact side sheet (21) and the non-skin contact side sheet (22) peel from each other when a shear force of 10 N or higher, preferably 18 N or higher, preferably 20 N or higher, is applied to the region by the rubbing, wherein the shear force is measured according to the method of the description.

6. The absorbent article according to any one of claims 1 to 5, wherein the region is designed such that the skin contact surface side sheet (21) and the non-skin contact surface side sheet (22) peel from each other when a shear force of 10 to 27 N, preferably 18 to 25 N, preferably 20 to 23 N, is applied to the region by the rubbing, wherein the shear force is measured according to the method of the description.

7. The absorbent article according to any one of claims 1 to 6, wherein the region where, when rubbed, the friction coefficient or the mean deviation of the friction coefficient decreases compared to before the rubbing has a friction coefficient MIU of 0.30 or less, preferably 0.27 or less, before the rubbing.

8. The absorbent article according to any one of claims 1 to 7, wherein the region where, when rubbed, the friction coefficient or the mean deviation of the friction coefficient decreases compared to before the rubbing has a friction coefficient MIU of 0.27 or less, preferably 0.25 or less, after the rubbing.

9. The absorbent article according to any one of claims 1 to 8, wherein the region where, when rubbed, the friction coefficient or the mean deviation of the friction coefficient decreases compared to before the rubbing has a ratio of the MIU after the rubbing to the MIU before the rubbing, MIU after rubbing/MIU before rubbing, of 0.95 or lower, preferably 0.90 or lower.

10. The absorbent article according to any one of claims 1 to 9, wherein the regions are arranged discretely on the skin contact side.

## Patentansprüche

1. Absorbierender Artikel, der auf einer Hautkontaktseite davon eine Lage (2) aufweist, die einen Bereich aufweist, in dem, wenn sie unter den nachstehenden Bedingungen gerieben wird, ein Reibungskoeffizient oder eine mittlere Abweichung des Reibungskoeffizienten um 5 % oder mehr bzw. 15 % oder mehr abnimmt, verglichen mit vor dem Reiben, und der eine Längsrichtung, die einer Richtung von vorn nach hinten eines Trägers entspricht, und eine laterale Richtung senkrecht zur Längsrichtung aufweist, wobei die Lage eine dreidimensional geformte Laminatlage ist, die aus einer hautkontaktseitigen Lage (21) und einer nicht-hautkontaktseitigen Lage (22) zusammengesetzt ist, wobei die hautkontaktseitige Lage (21) und die nicht-hautkontaktseitige Lage (22) an mehreren Verbindungen (23) miteinander verbunden sind, die hautkontaktseitige Lage (21) mehrere Vorsprünge (24) bildet, die von der nicht-hautkontaktseitigen Lage (22) weg in anderen Abschnitten als den Verbindungen (23) vorstehen, und der Bereich so gestaltet ist, dass sich die hautkontaktseitige Lage (21) und die nicht-hautkontaktseitige Lage (22) beim Reiben an der Verbindung (23) voneinander ablösen, wobei die Bindung (23) in dem Bereich Abschnitte mit unterschiedlichen Dicken aufweist, und die Ablösung zwischen der hautkontaktseitigen Lage (21) und der nicht-hautkontaktseitigen Lage (22) so gestaltet ist, dass sie durch das Reiben zwischen den Abschnitten mit unterschiedlichen Dicken eingeleitet wird:
Bedingungen: ein nasser Kollagenfilm wird 10 Mal auf der Lage (2) in der Längsrichtung der Lage (2) unter einer Last von 3,0 kPa hin- und herbewegt; und der nasse Kollagenfilm weist einen Wassergehalt von 100 bis 150 Masse-% auf, bezogen auf sein Trockengewicht, und wird durch Eintauchen eines Kollagenfilms in ionenausgetauschtes Wasser für 1 Minute, Herausnehmen des Kollagenfilms und Entfernen von überschüssigem Wasser vorbereitet;
wobei der Reibungskoeffizient und die mittlere Abweichung des Reibungskoeffizienten gemäß der Methode der Beschreibung gemessen werden.

2. Absorbierender Artikel nach Anspruch 1, wobei die Lage (2) eine flüssigkeitsdurchlässige Oberlage (2) ist, die die Hautkontaktseite definiert, und der Bereich in mindestens einem lateral mittleren Abschnitt der Oberlage (2) vorhanden ist.

3. Absorbierender Artikel nach Anspruch 1 oder 2, wobei die Bindung (23) in dem Bereich eine nach unten konvexe Form im Querschnitt in Dickenrichtung aufweist und Fasern, die die Schicht (22) auf der Nicht-Hautkontaktseite bilden, bereit sind, um die Bindung durchtrennt zu werden, wenn sie gerieben werden.

4. Absorbierender Artikel nach Anspruch 1 oder 2, wobei die Bindung (23) in dem Bereich eine nach oben konvexe Form im Querschnitt in der Dickenrichtung aufweist und Fasern, die die hautkontaktseitige Lage (21) bilden, bereit sind, um die Bindung durchtrennt zu werden, wenn sie gerieben werden.

5. Absorbierender Artikel nach einem der Ansprüche 1 bis 4, wobei der Bereich so gestaltet ist, dass sich die hautkontaktseitige Lage (21) und die nicht-hautkontaktseitige Lage (22) voneinander ablösen, wenn eine Scherkraft von 10 N oder höher, vorzugsweise 18 N oder höher, vorzugsweise 20 N oder höher, durch das Reiben auf den Bereich ausgeübt wird, wobei die Scherkraft gemäß dem Verfahren der Beschreibung gemessen wird.

6. Absorbierender Artikel nach einem der Ansprüche 1 bis 5, wobei der Bereich so gestaltet ist, dass sich die hautkontaktseitige Lage (21) und die nicht-hautkontaktseitige Lage (22) voneinander ablösen, wenn eine Scherkraft von 10 bis 27 N, vorzugsweise 18 bis 25 N, vorzugsweise 20 bis 23 N, durch das Reiben auf den Bereich ausgeübt wird, wobei die Scherkraft nach dem Verfahren der Beschreibung gemessen wird.

7. Absorbierender Artikel nach einem der Ansprüche 1 bis 6, wobei der Bereich, in dem beim Reiben der Reibungskoeffizient oder die mittlere Abweichung des Reibungskoeffizienten im Vergleich zu vor dem Reiben abnimmt, vor dem Reiben einen Reibungskoeffizienten MIU von 0,30 oder weniger, vorzugsweise 0,27 oder weniger, aufweist.

8. Absorbierender Artikel nach einem der Ansprüche 1 bis 7, wobei der Bereich, in dem, wenn er gerieben wird, der Reibungskoeffizient oder die mittlere Abweichung des Reibungskoeffizienten im Vergleich zu vor dem Reiben abnimmt, nach dem Reiben einen Reibungskoeffizienten MIU von 0,27 oder weniger, vorzugsweise von 0,25 oder weniger, aufweist.

9. Absorbierender Artikel nach einem der Ansprüche 1 bis 8, wobei der Bereich, in dem, wenn er gerieben wird, der Reibungskoeffizient oder die mittlere Abweichung des Reibungskoeffizienten im Vergleich zu vor dem Reiben abnimmt, ein Verhältnis des MIU nach dem Reiben zum MIU vor dem Reiben, MIU nach dem Reiben/MIU vor dem Reiben, von 0,95 oder weniger, vorzugsweise 0,90 oder weniger, aufweist.

10. Absorbierender Artikel nach einem der Ansprüche 1 bis 9, wobei die Bereiche diskret auf der Hautkontaktseite angeordnet sind.

## Revendications

1. Article absorbant comprenant, sur sa face en contact avec la peau, une feuille (2) présentant une zone où, lorsqu'elle est frottée dans les conditions ci-dessous, un coefficient de frottement ou un écart moyen du coefficient de frottement décroissent de 5 % ou plus ou de 15 % ou plus, respectivement, par rapport à ce qu'il étaient avant frottement, et ayant une direction longitudinale correspondant à la direction avant-arrière d'un porteur et une direction latérale perpendiculaire à la direction longitudinale, où la feuille est une feuille stratifiée de forme tridimensionnelle composée d'une feuille latérale en contact avec la peau (21) et d'une feuille latérale exempte de contact avec la peau (22), la feuille latérale en contact avec la peau (21) et la feuille latérale exempte de contact avec la peau (22) étant reliées l'une à l'autre par une pluralité de liaisons (23), la feuille latérale en contact avec la peau (21) formant une pluralité de saillies (24) s'éloignant de la feuille latérale exempte de contact avec la peau (22) dans des parties autres que les liaisons (23), et la zone étant conçue de sorte que la feuille latérale en contact avec la peau (21) et la feuille latérale exempte de contact avec la peau (22) se détachent l'une de l'autre au niveau de la liaison (23) lorsqu'elles sont frottées, où la liaison (23) dans la zone comprend des parties d'épaisseurs différentes, et la séparation par pelage entre la feuille latérale en contact avec la peau (21) et la feuille latérale exempte de contact avec la peau (22) est conçue pour être amorcée entre les parties d'épaisseurs différentes sous l'effet du frottement :
conditions : un film de collagène humide est déplacé en va-et-vient 10 fois sur la feuille (2) dans la direction longitudinale de la feuille (2) sous une charge de 3,0 kPa ; et le film de collagène humide a une teneur en eau de 100 à 150 % en masse par rapport à son poids à sec et est préparé par immersion d'un film de collagène dans de l'eau soumise à échange ionique pendant 1 minute, retrait du film de collagène et élimination de l'excès d'eau ;
où le coefficient de frottement et l'écart moyen du coefficient de frottement sont mesurés conformément au procédé de la description.

2. Article absorbant selon la revendication 1, où la feuille (2) est une feuille supérieure perméable aux liquides (2) définissant le côté en contact avec la peau, et où la zone est présente dans au moins une partie centrale latérale de la feuille supérieure (2).

3. Article absorbant selon la revendication 1 ou la revendication 2, où la liaison (23) dans la zone a une forme convexe vers le bas en section transversale dans le sens de l'épaisseur, et les fibres constituant la feuille latérale exempte de contact avec la peau (22) sont prêtes à être sectionnées autour de la liaison lorsqu'elles sont frottées.

4. Article absorbant selon la revendication 1 ou la revendication 2, où la liaison (23) dans la zone a une forme convexe vers le haut en section transversale dans le sens de l'épaisseur, et les fibres constituant la feuille latérale en contact avec la peau (21) sont prêtes à être sectionnées autour de la liaison lorsqu'elles sont frottées.

5. Article absorbant selon l'une des revendications 1 à 4, où la zone est conçue de sorte que la feuille latérale en contact avec la peau (21) et la feuille latérale exempte de contact avec la peau (22) se détachent l'une de l'autre lorsqu'une force de cisaillement de 10 N ou plus, avantageusement de 18 N ou plus, de préférence de 20 N ou plus, est appliquée sur la zone par frottement, ladite force de cisaillement étant mesurée conformément au procédé de la description.

6. Article absorbant selon l'une des revendications 1 à 5, où la zone est conçue de sorte que la feuille latérale (21) en contact avec la peau et la feuille latérale (22) exempte de contact avec la peau se détachent l'une de l'autre lorsqu'une force de cisaillement de 10 à 27 N, avantageusement de 18 à 25 N, de préférence de 20 à 23 N, est appliquée sur la zone par frottement, ladite force de cisaillement étant mesurée conformément au procédé de la description.

7. Article absorbant selon l'une des revendications 1 à 6, où la zone où, lors du frottement, le coefficient de frottement ou l'écart moyen du coefficient de frottement décroissent par rapport à ce qu'il étaient avant frottement, présente un coefficient de frottement MIU de 0,30 ou moins, de préférence de 0,27 ou moins, avant frottement.

8. Article absorbant selon l'une des revendications 1 à 7, où la zone où, lors du frottement, le coefficient de frottement ou l'écart moyen du coefficient de frottement décroissent par rapport à ce qu'il étaient avant frottement, présente un coefficient de frottement MIU de 0,27 ou moins, de préférence de 0,25 ou moins, après frottement.

9. Article absorbant selon l'une des revendications 1 à 8, où la zone où, lors du frottement, le coefficient de frottement ou l'écart moyen du coefficient de frottement décroissent par rapport à ce qu'il étaient avant frottement, présente un rapport entre le MIU après frottement et le MIU avant frottement, MIU après frottement/MIU avant frottement, de 0,95 ou moins, de préférence de 0,90 ou moins.

10. Article absorbant selon l'une des revendications 1 à 9, où les zones sont disposées discrètement sur la face en contact avec la peau.
